# EUROPEAN PATENT APPLICATION

(11) **EP 4 635 424 A1**
(43) Date of publication of application: **22.10.2025**
(21) Application number: 24315178.4
(22) Date of filing: 16.04.2024
(51) Int. Cl.: A61B 17/00, A61B 17/04

(54) **A CATHETER DEVICE, A SUTURE, AND A METHOD OF SUTURING, AND A SUTURE ACTUATOR**

(71) Applicant: HoliStick Medical, 75013 Paris (FR)
(72) Inventor: The designation of the inventor has not yet been filed
(74) Representative: Hepp Wenger Ryffel AG

(57) **Abstract**

The invention is directed to a catheter device (1) for delivering a suture (21) to a region of a defect in a tissue. The catheter device (1) comprises a proximal end (3) and a distal end (2) and at least a first lumen (4) extending from the proximal end (3) toward the distal end (2). The first lumen (4) is adapted for receiving a suture (21). The catheter device (1) further comprises, or is connectable to, a suture actuator (10) adapted to move the suture (21), when arranged in the lumen (4), in a direction from proximal to distal.

## Description

The present invention relates to a catheter device, a suture, and a method a according to the preamble of the independent claims.

There are several tissue openings in the human or animal bodies that may need to be closed to treat certain conditions. For example, atrial septal defects such as the patent foramen ovale (PFO) can cause a variety of symptom which can be treated by closing said defect.

Several devices and methods are known in the prior art to close tissue openings.

CN 212394986 discloses a suturing device which is used for minimally invasive surgical suturing.

US 7,846,179 discloses a system for treating a septal defect having a suture-like implantable treatment apparatus, devices for delivering the implantable treatment apparatus and methods for treating a septal defect. The suture-like apparatus is implantable through a septal wall or portion thereof.

US 6,911,034 discloses a suturing apparatus including a needle which is adapted to pierce the inner surface of a biological structure.

US 8,246,636 discloses methods and apparatuses for closing a patent foramen ovale. First and second suture clasp arms are adapted to hold end portions of a suture when in an extended position, and a first suture catch mechanism and a second suture catch mechanism are present.

EP 1 236 437 A1 discloses a suture applying device comprising a shaft which carries a pair of needles near its distal end. The needles are joined by a length of suture. After the needles have passed through the tissue, a loop of suture is left being to close the puncture site near the body lumen.

US 2012/296373 A1 discloses a closure device for closing an opening in tissue. The closure device includes an elongate member through which needles may be deployed.

US 2009/306685 A1 discloses a noninvasive trans-catheter method and apparatus for remote suture placement can be used in inner cardiac applications.

However, known devices and methods are burdensome for operators and deployment of sutures may require extensive manipulation of devices.

Thus, the object of the present invention is to overcome the drawbacks of the prior art, in particular to provide a reliable, simple, and effective way of deploying sutures from needles.

This and other objects are achieved by the catheter device, the suture, the methods, and the suture actuator according to the characterizing portion of the independent claims of the invention.

The invention is directed to a catheter device for delivering a suture to a region of a defect in a tissue, in particular to an atrial or septal defect. The catheter device comprises a proximal end and a distal end, and at least a first lumen extending from the proximal end to the distal end. The lumen may be elongated needle and the distal end a needle tip. The first lumen is adapted for receiving a suture, e.g. a suture may be at least partially arranged in the lumen. The catheter device may further comprise and/or be connectable to a suture actuator. The suture actuator is adapted to move the suture, when the suture is arranged in the lumen. The movement of the suture may be from proximal to distal, e.g. such as to push the suture out the needle tip.

The suture actuator enable a simple and effective movement of the suture out of the distal end of the lumen. For example, when the lumen comprises a needle tip to pierce a tissue, the suture may be ejected after piercing the tissue. As a result, after retraction of the needle, the suture may remain in the tissue.

Furthermore, pushing of a suture may cause the suture to kink. The kink may cause additional friction in a needle and prevent safe ejection.

The catheter device may comprise at least a second lumen extending from the proximal end toward the distal end, and further comprising a first needle connected or connectable to the first lumen and a second needle connected or connectable to the second lumen. Alternatively, the first and/or the second lumen may be formed by an elongated needle. A first and a second channel are formed for delivery of the suture. The catheter device comprises an expandable proximal tissue support adapted to push, in a proximal direction, against a tissue wall to be pierced.

It will be understood that the device may comprise two, three, or four identically configured suture actuators each connected or connectable to a lumen and/or a needle.

The catheter device may comprise at least one fluid reservoir. The fluid reservoir may be pre-loaded with a suture. The fluid reservoir may be in operable connection with a mechanism for injecting fluid from the fluid reservoir into the first and/or the second lumen. The suture may extend from the fluid reservoir into the first and/or the second lumen. By the injection of the fluid, the suture may be propelled into the first and/or the second lumen and moved thereby.

For example, the mechanism for injecting the fluid may be piston for reducing the volume of the fluid reservoir and/or a pressure line for increasing the pressure in the fluid reservoir. The fluid reservoir may be connected to the lumen.

Thus, a suture which is arranged in the lumen and/or pre-loaded in the fluid reservoir can be ejected with a simple mechanism.

Furthermore, the first and/or second lumen may have a crosssection area which is filled to at least 50 %, preferably at least 80%, by a crossectional area of the suture. As a result, only small volumes of fluid are necessary to achieve high fluid flow velocity and corresponding movement of the suture. On the one hand, this leads to an efficient ejection of sutures which may not require extensive manipulation by an operator. On the other hand, the amount of fluid which is injected into the patient for suture ejection is minimized.

The fluid reservoir may be arranged at the proximal end of the catheter device, in particular in a handle of the catheter device.

The fluid reservoir and/or the mechanism for injecting fluid may be formed by a syringe and a piston.

The syringe may be a commercially available syringe which may provide a particularly simple and versatile fluid reservoir and fluid injection. However, it will be understood that syringe shall be understood, in the context of the present invention, as any

The suture actuator may be at least partially formed by a rack gear pusher.

The suture actuator may be at least partially formed by at least one roll.

The suture actuator may at least partially be formed by a wire pusher connected or connectable to the suture. The connection between suture and wire pusher may be formed at a distal end of the wire pusher.

The suture actuator may be at least partially formed by a gripper. The gripper may, preferably, be formed by a compressible hypotube. The gripper may be slidably arranged within the first lumen and/or the second lumen and adapted to hold the first and/or the second suture.

The suture actuator may at least partially be formed by a magnetic actuator. The magnetic actuator may be adapted to interact, preferably selectively, with a magnetic element arranged on the suture.

The suture actuator may at least partially be formed by a telescope mechanism.

The catheter device may comprise a suture and a spool adapted to release the suture in response to the suture actuator being actuated.

The suture may be coiled around the spool. The spool may be arranged in the fluid reservoir and rotatable around an axis. The spool may be allowed to rotate freely, e.g. such that when the suture is pulled, the suture is automatically released.

This may prevent entangling of the suture and thus provide a particularly safe treatment.

The invention is further directed to a suture for use in a catheter device as described herein. The suture comprises at least one of a magnetic element, a flare, and a high-friction surface, for example a hydrogel.

These elements provide enhanced engagement with a suture actuator and thus further improve the suture ejection.

The suture may comprise a flat ribbon section. At least one marker may be arranged on a first and/or flat end of the flat ribbon section.

A flat ribbon section may be less traumatic than a standard surgical sutures. Thus, the flat ribbon section may be placed at the tissue to be treated to avoid injuries from movement of the suture. The marker may help an operator in localizing the flat ribbon section and thus assist in placing the ribbon section appropriately.

For example, the flat ribbon section may comprise or consist of expanded polytetrafluoroethylene (PTFE).

The marker may be one of a color marker and a radio-opaque marker.

The invention is further directed to a method of suturing a tissue, in particular a heart tissue. The method may be performed minimally invasively. The method comprises piercing the tissue with at least one needle forming part of a catheter device. The catheter device may be a catheter device as described herein. A suture is ejected from the needle, e.g. in distal direction and out of a needle tip, by means of a fluid flow.

The needle may be retracted while the suture may remain in the tissue. The suture may also be pulled back through another opening, e.g. a patent foramen ovale or another pierced hole, to form a loop through the tissue.

The suture may be captured, after ejection, using an expandable suture-capture mesh.

The invention is further directed to a suture actuator comprising a fluid reservoir with a pre-loaded suture. The suture actuator may be adapted for use with a catheter device as described herein.

In the following, the invention is described in detail with reference to the following figures, showing:
- Fig. 1:: a first embodiment of a suture actuator.
- Fig. 2:: the first embodiment of the suture actuator when assembled.
- Fig. 3:: a second embodiment of a suture actuator.
- Figs. 4a-4b:: a detailed view of portion of the device of Fig. 3.
- Fig. 5:: a third embodiment of a suture actuator.
- Fig. 6a-6b:: a fourth embodiment of a suture actuator.
- Fig. 7:: a suture holder body.
- Fig. 8:: a fifth embodiment of a suture actuator.
- Fig. 9:: a sixth embodiment of a suture actuator.
- Fig. 10-10b:: an seventh embodiment of a suture actuator.
- Fig. 11:: a eighth embodiment of a suture actuator.
- Fig. 12:: a ninth embodiment of a suture actuator.
- Fig. 13:: another view of the embodiment of Fig. 12.
- Fig. 14:: a tenth embodiment of a suture actuator.
- Fig. 15a-15b:: a eleventh embodiment of a suture actuator.
- Figs. 16a-16b:: a twelfth embodiment of a suture actuator.
- Fig. 17:: a first embodiment of a suture.
- Fig. 18:: a second embodiment of a suture.
- Fig. 19:: schematically a catheter device.
- Fig. 20a-20b:: a handle of a catheter device.
- Fig. 21:: schematically a first embodiment of a catheter device in use.
- Fig. 22:: schematically a second embodiment of a catheter device in use.

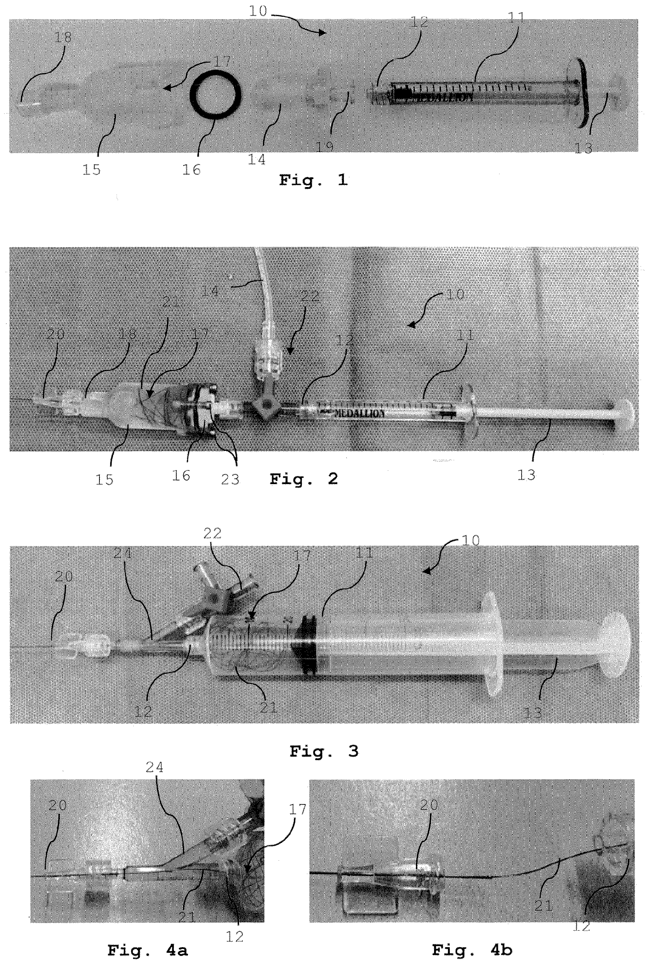

Fig. 1 shows a suture actuator 10 according to the invention in a disassembled state. The actuator comprises a syringe 11 with a female Luer lock connector 12 and a piston 13. A fluid chamber 17 is formed by a silicone cup 15 and a plug 14 which are connectable. A O-ring 16 may be arranged therebetween for increased sealing. The cup 15 comprises a second female Luer lock connector 18. Actuation of the piston 13 in the syringe 11 pushes fluid out of the syringe 11, through channel 19 into the fluid channel 17. A suture (not shown) may be arranged in the fluid chamber 17.

Fig. 2 shows a suture actuator 10 similar to the embodiment of Fig. 1. For clarity, corresponding features with the same reference numerals are not described repeatedly. Here, an elongate needle 20 is connected to the female Luer lock connector 18. The needle may form a lumen in a catheter device (not shown). Furthermore, a suture 21 is arranged in the fluid chamber 17 and extends into needle 21 via Luer lock connector 18. A T-connector 22 is arranged between the syringe 11 and the fluid chamber assembly 15, 16, 17. It will be noted that the plug 16 is attached to the cup 15 with screws 23 for additional safety. A fluid line 14 is connected to the T-connector 22. The T-connector 22 allows to selectively create a fluid communication channel between the syringe 11 and the fluid chamber 17 to allow injection of fluid into the fluid chamber; between the syringe 11, the fluid line 14, and the fluid chamber 17 to allow fluid injection from both the syringe 11 and the fluid line 14 into the fluid chamber 17; or to close off the fluid chamber 17 from both the fluid line 14 and the syringe 11, whereby the fluid line 14 and the syringe may be closed off from one another or in fluid communication, e.g. for refilling of the syringe via fluid line 14.

Fig. 3 shows an embodiment of a suture actuator 10. Here, the syringe 11 forms the fluid chamber 17. A suture 21 is arranged in the fluid chamber 17. A y-connector 24 connects a needle 20 with the syringe 11. A T-connector 22 is connected to a second end of the Y-connector for selective injection of other fluids, though it will be understood that the T-connector 22 is not required for the suture actuator 10 to function and merely offers additional advantages in certain applications. By pushing the piston 13, the suture 21 is moved out of the fluid chamber 17 into needle 20 by the created fluid flow.

Fig. 4a shows a details view of the area of Y-connector 24 shown in Fig. 3. The suture 21 extending from the fluid chamber 17 into the connector is visible.

Fig. 4b shows the same area as Fig. 4a but with the Y-connector removed. The suture 21 is visible extending into the needle 20. It will be understood that the needle 20 may be connected directly to the female Luer lock connector 12 if desired.

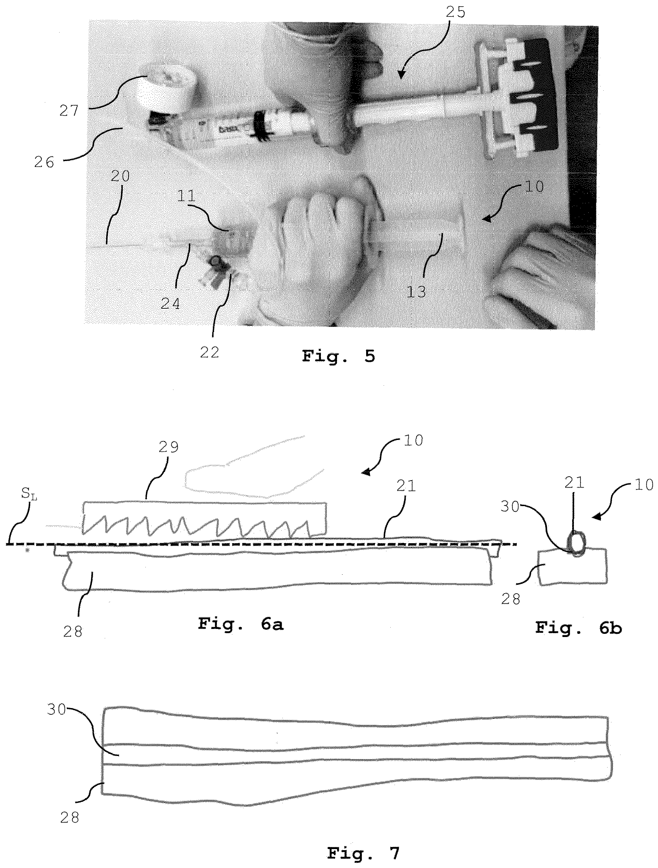

Fig. 5 shows the suture actuator 10 of Fig. 4, wherein a fluid supply 25 is connected, via a fluid line 26, to the T-connector 22. Furthermore, a barometer 27 is arranged at the fluid supply 25. When the T-connector 22 is set to create a fluid communication channel between the Y-connector 24 and the fluid supply 25, the pressure in the suture actuator may be measured. In addition, further fluid may be provided.

Fig. 6a shows an alternative embodiment of a suture actuator 10 in a side view. The suture 21 is slidably arranged, along suture axis S_{L}, on a suture holder 28. A rack gear pusher 29 is used to push the suture in a direction of ejection. Here, the suture 21 is pushed in a direction S_{L} by the rack gear pusher 29 after an engagement step, after which the suture 21 may be disengaged again.

Fig. 6b shows a cross-sectional view of the embodiment of the suture actuator 10 of Fig. 6a in a plane perpendicular to the suture axis. The suture 21 is arranged in a groove 30 of suture holding.

Fig. 7 shows the suture holder 28 of Figs. 6a-6b with groove 30 in a top view.

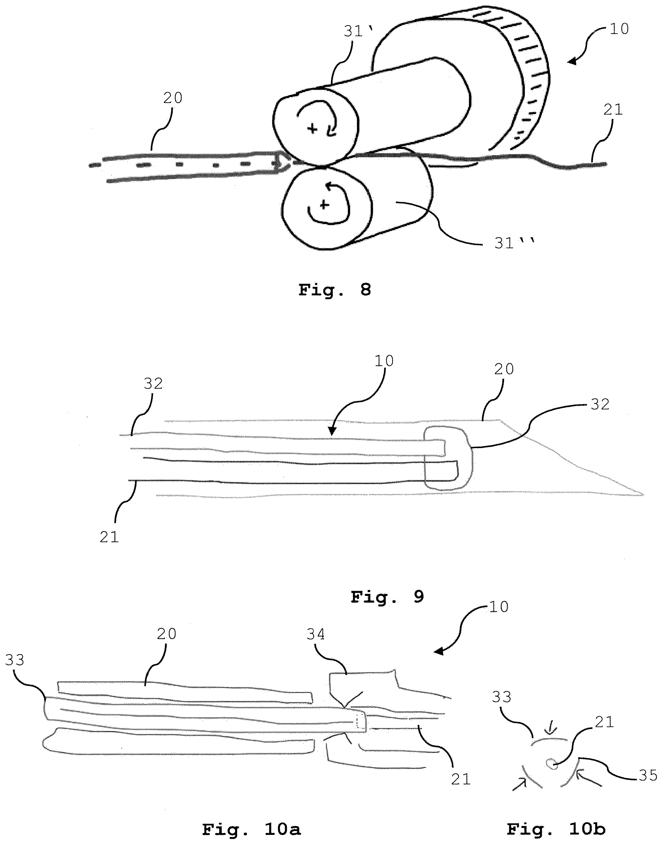

Fig. 8 shows a suture actuator 10 comprising a first roll 31' and a second roll 31" which are in frictional engagement with suture 21. Rolling of rolls 31', 31" causes the suture to be moved into needle 20.

Fig. 9 shows a further embodiment of a suture actuator 10. Here, the suture 21 is placed in a needle 20. The suture 21 is actuated by a pusher wire 32 which is equipped with a magnetic connector 32 holding the suture 21 at a distal end, via a magnetic distal end of the suture.

Fig. 10 shows a suture actuator 10 comprising a hypotube 33 arranged concentrically around a suture 21 within a needle 20. The hypotube 33 is slidably arranged within the needle 20. A pincher 34 is arranged at a proximal end of the hypotube 33 and adapted to radially compress the hypotube 33 to grip the suture 21. When compressed, movement of the hypotube 33 along the needle 20 moves the suture 21 along. To do so, the pincher 34 may also be axially movable. The pincher 34 thus cause gripping of the suture 21, via hypotube 33, for distal movement. Subsequently, the suture 21 may be released, the pincher 34 may be moved back proximally to a repeated gripping and moving step.

Fig. 10b shows a cross-sectional view of the hypotube 22 with the suture 21 arranged therein. The needle and pincher (see Fig. 10a) are not shown for clarity. The hypotube 33 comprises three slits 35 which allow a compression, when pinched, to grip the suture 21.

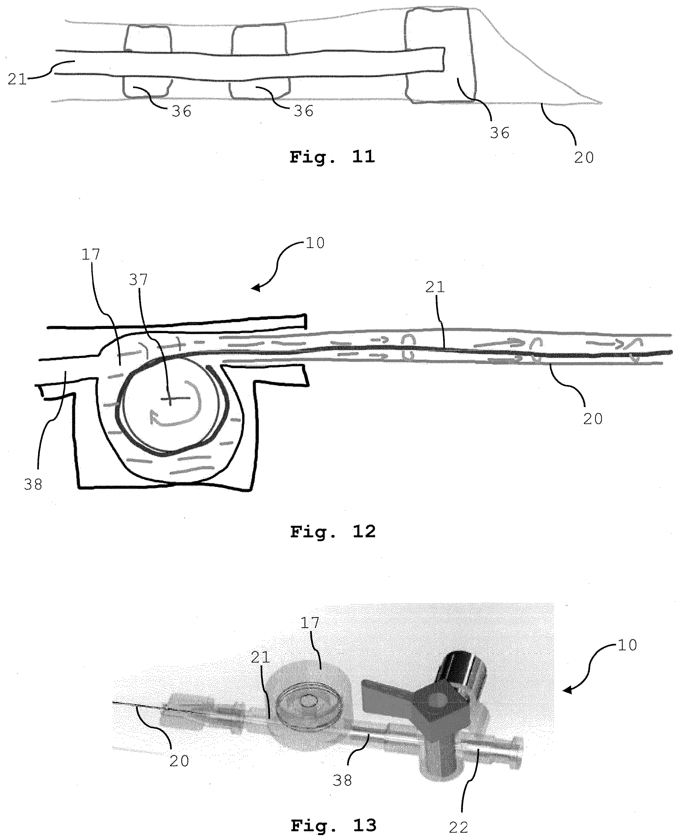

Fig. 11 shows a needle 20 with a suture 21 according to the invention arranged therein. The suture 21 comprises three umbrellas 36 arranged at a distal end and extending away from the suture axis. The umbrellas 36 increase the flow resistance, in a direction of the suture axis, so as to increase a propelling force exerted by a fluid flow (e.g. due to a suture actuator of Figs. 1-5). Additionally or alternatively to umbrellas 36, magnetic elements may be arranged on the suture.

Fig. 12 shows a suture actuator 10 which is similar to the embodiment of Fig. 1. Here, a spool 37 is arranged within the fluid chamber 17 and the suture 21 is coiled on the spool 37. A syringe 11 or other means for injecting fluid may be arranged in fluid communication with port 38. When fluid is injected, the flow of fluid through needle 20 pulls the suture 21 off the spool 37.

Fig. 13 shows a similar suture actuator as Fig. 12. Here, a T-connector is additionally connected upstream from the fluid chamber 17 with spool 17.

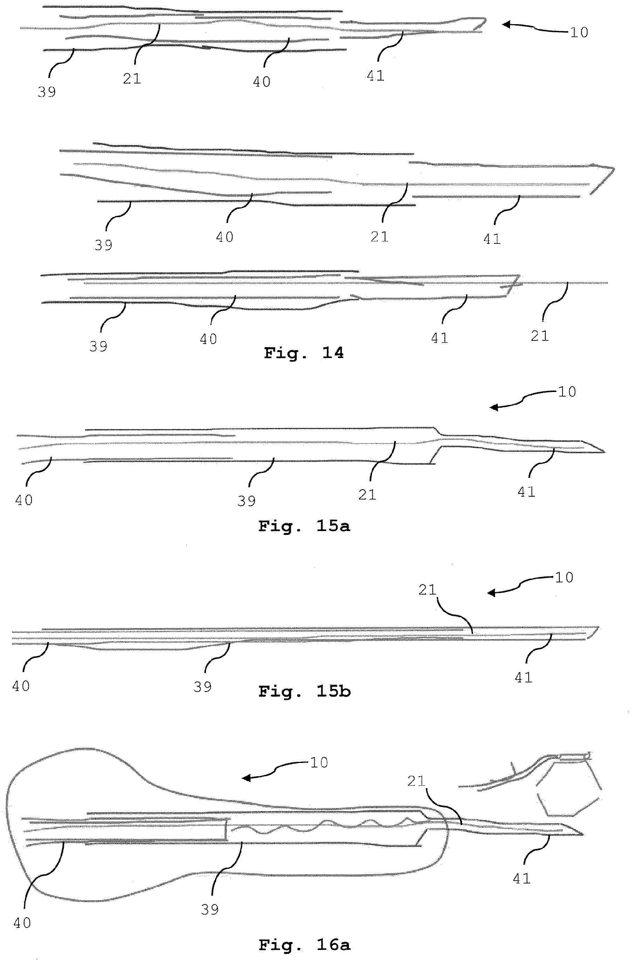

Fig. 14 shows a suture actuator 10 having a first tube 39 and second tube 40, and a connector 41. The connector 41 is adapted to releasably connect to the suture. The second tube 40 is telescopically arranged within the first tube 39 and may prevent kinking of the suture when it is pushed. As shown in the middle panel, the connector 41 may be pushed forward, thereby pulling the suture 21 along. Optionally, the first tube 39 is moved with the connector 41, whereas the second tube 40 remains in place. Due to the movement of the first tube 40, the overall friction between suture 21 and inner wall of the tubes 39, 40 may be reduced. As shown in the bottom panel, the connector 41 may be moved back after releasing the suture 21, so that the suture 21 remains in its moved position and extends out of connector 41. The suture 21 may then be moved again by repetition of the steps described herein.

Fig. 15a shows an alternative embodiment which is similar to the telescope assembly shown in Fig. 14. Here, the connector 41 is integrally formed with the first tube 39 and has a smaller cross-section than the first tube 39.

Fig. 15b shows a similar embodiment to the suture actuator 10 of Fig. 15a. here, the connector 41 consists of fixed connection of suture 21 at a distal end of first tube 39.

Fig. 16a shows a similar embodiment of a suture actuator 10 as Figs. 14-15, having a telescopic tube assembly 39, 40.

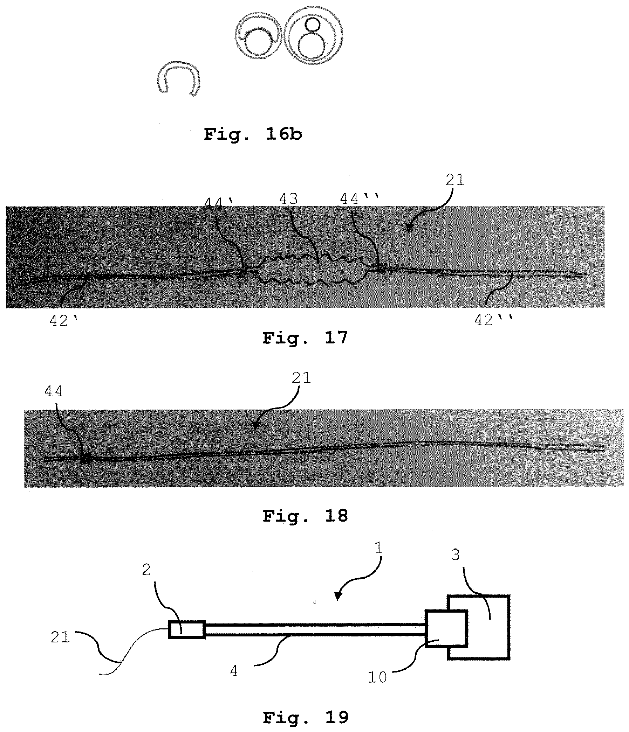

Fig. 16b shows cross-sectional views of the embodiment of Fig. 16a.

Fig. 17 shows a suture 21 according to the invention. The suture 21 has a first section 42' and a second section 42" which essentially have a circular cross-section. Between section 42' and section 42'', a flat ribbon 43 made of expanded PTFE is arranged. The flat ribbon 43 is less traumatic to tissue than sections 42',42". On each side of the flat ribbon 43, a radio-opaque marker 44', 44" is arranged. Thus, when the suture 21 is deployed, an operator can identify the section of the suture 21 corresponding to the flat ribbon 43 for accurate placement.

In a similar fashion, it would be conceivable to use a suture 21 without a flat ribbon 43 but still use markers 44', 44'' to determine a position of a suture. The markers may also, additionally or alternatively, comprise or consist of color codes or similar indications which allow a user to determine how much suture has been advanced through the device. Preferably, such markings are arranged along the entire length of the suture. For example, a radio-opaque marker and/or a colored section may be arranged every 5 cm along the suture.

Fig. 18 shows an alternative embodiment of a suture 21 having one radio-opaque marker 44 along its length. It will be understood that this embodiment, though not shown, may be combined with a ribbon (see Fig. 17).

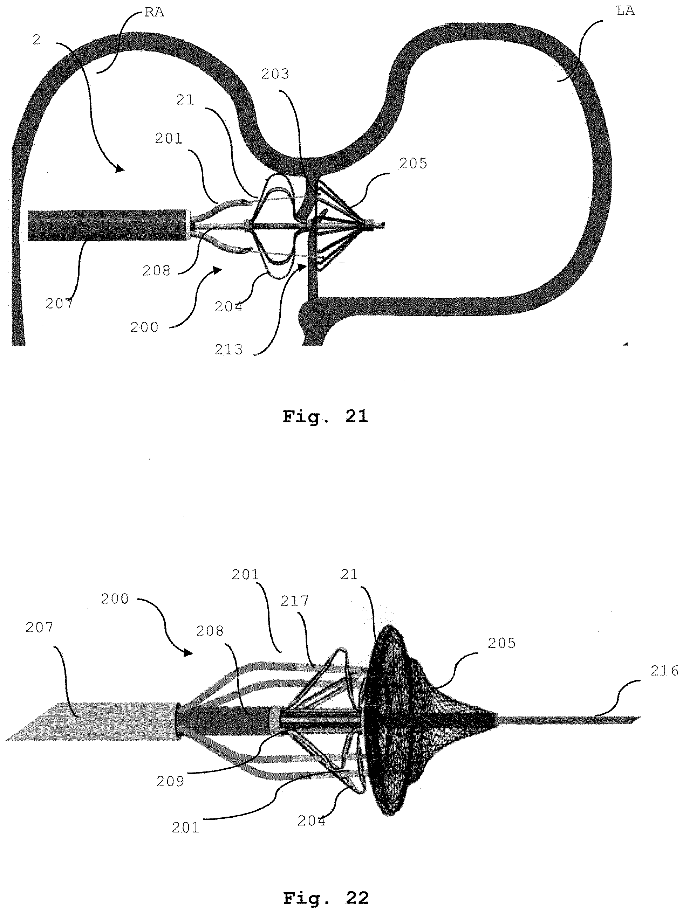

Fig. 19 shows, schematically, a catheter device 1 according to the invention. The catheter device 1 comprises a distal portion 2adapted for suturing tissue (see, e.g. Figs. 21 and 22), a handle 3 in operable connection with a suture actuator 10, and a lumen 4. A suture extends through lumen 4 and out of the distal portion 2. It will be understood that the suture actuator 10 may be any suture actuator as shown in Figs. 1-16, and may also be arranged along the lumen 4 or in proximity to the distal portion 2.

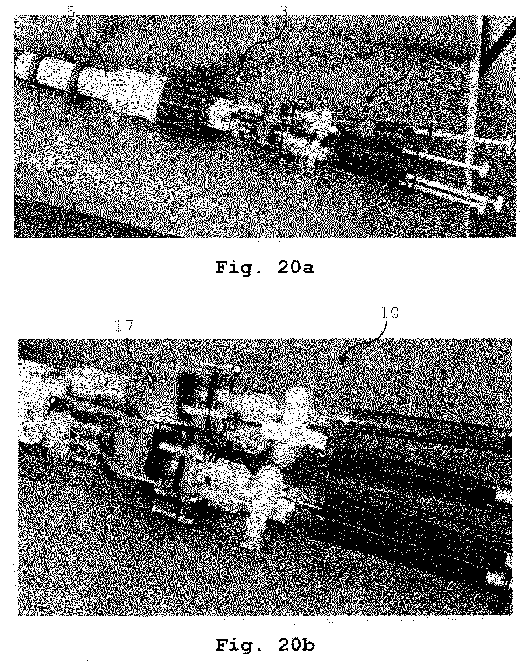

Fig. 20a shows a proximal portion 3 of a catheter device 1 having a handle 5. The catheter device 1 comprises four suture actuators 10 substantially corresponding to the embodiment shown in Fig. 2. Here, the suture actuators 10 are each directly connected to a lumen (not visible, see Fig. 19) of the catheter device 1. The catheter device 1 has four separate lumens each connected to a needle at a distal end (not shown, see Figs. 21 and 22) .

Fig. 20b shows a detailed view of the suture actuators 10 of the catheter device 1 of Fig. 20a.

Figs. 21 and 22 show schematically two alternative concepts of a distal portion 2 of a catheter device (see Fig. 19) configured as a suturing device 200. It will be understood that the distal portions 2 shown here may be combined with any suture actuator described herein to form a catheter device.

The suturing device 200 is used to treat an atrial septal defect and is deployed therethrough, from the right atrium RA into the left atrium. A shaft 207 remains in the right atrium and deployable needles 201 are used to close the defect using multiple sutures 21.

Fig. 21 shows a suturing device 200 which deploys needles 201 from a proximal direction to pierce in a distal direction, here from right atrium RA into the left atrium LA. To this end, the device 200 comprises a distal tissue support 205 which provides a counter force toward a proximal direction, on a distal side of the tissue to be pierced, in the left atrium LA. The needles 201 are arranged within and deployed from the shaft 207. Along a central axis, the inner shaft 208 extends on which, at a distal end, the tissue support 205 is arranged. In addition, the device 200 shown here has proximal support 204 which provides additional tissue support to more securely pierce the tissue. It will be understood that a proximal anchor 204 is optional in this embodiment. Both tissue supports 204, 205 are configured as expandable wires which may be delivered in an extended elongated shape in which the wires are substantially parallel to the inner shaft 208, and deployed into an arc shape as shown here. Here, the sutures 21 are connected to pledgets 203 to secure the suture to the tissue wall 213.

Fig. 22 shows a suturing device 200 similar, in its functionality, to the device shown in Fig. 21. Here, needles 201 extend from within the shaft in a distal direction. In the shown configuration, the needles 201 are deployed from the shaft 207. A distal tissue support 205 is arranged on an inner-most shaft 209 and a proximal support 204 is arranged on an intermediate shaft 208. The fuctionalities of the proximal support 204 and the distal support 205 substantially corresponds to the functionalities described above in the context of Fig. 21. However, in addition, the distal support 205 is further configured as a mesh. In a fully expanded state, as shown here, the needles 201 can extend through the mesh. A suture 21 which is ejected from the needles 201 can be temporarily fixed, by being pinched, to the mesh when the proximal support is compressed at least partially. Thus, this present embodiment is particularly suitable when no pledgets (see Fig. 21) are used to fix the sutures 21 to the tissue. Instead, with the present embodiment, the suture 21 may be delivered through the tissue by piercing and held by the distal support 205 while the needles 201 are retracted back through the tissue. The distal support 205 may then be pulled back through the tissue opening, and bring the sutures 21 back toward the device 200. Another end of the sutures 21 may still be held by the needles 201. As a result, the tissue is held be sutures 202 which may be fastened on one side of the tissue without requiring pledgets.

Here, the device 200 further comprises needle holders 217 attached to the proximal support 204. As a result, the needles 201 are automatically moved in a direction perpendicular to the longitudinal axis, away from the said axis, when the proximal support 204 is deployed. It will be understood that needle holders 217 are not required for this particular embodiment and may be combined with any other embodiment shown herein. The combination shown here is for exemplary purposes only. Finally, the device 200 shown here further has an inner channel for a guidewire 216.

## Claims

1. A catheter device (1) for delivering a suture (21) to a region of a defect in a tissue, comprising a proximal end (3) and a distal end (2) and at least a first lumen (4) extending from the proximal end (3) toward the distal end (2), wherein the first lumen (4) is adapted for receiving a suture (21),
**characterized in that**
the catheter device (1) further comprises, or is connectable to, a suture actuator (10) adapted to move the suture (21), when arranged in the lumen (4), in a direction from proximal to distal.

2. The catheter device (1) according to claim 1, comprising at least a second lumen (4) extending from the proximal end (3) toward the distal end (2), and further comprising a first needle (20, 201) connected or connectable to the first lumen (4) and a second needle (20, 201) connected or connectable to the second lumen (4), so as to form a first and a second channel for delivery of the suture (21), and further comprising an expandable proximal tissue support (204) adapted to push, in a proximal direction, against a tissue wall to be pierced.

3. The catheter device (1) according to any one of the preceding claims, further comprising at least one fluid reservoir, preferably with a pre-loaded suture (21), and being in operable connection with a mechanism for injecting fluid from the fluid reservoir into the first, and preferably the second, lumen such as to propel the suture (21) into the first, and preferably the second lumen.

4. The catheter device (1) according to claim 3, wherein the fluid reservoir is arranged at the proximal end (3) of the catheter device (1), preferably in a handle (5).

5. The catheter device (1) according to claim 4, wherein the fluid reservoir (17) and/or the mechanism for injecting fluid are formed by a syringe (11) and a piston (13).

6. The catheter device (1) according to any one of the preceding claims, wherein the suture actuator (10) is at least partially formed by a rack gear pusher (29).

7. The catheter device (1) according to any one of the preceding claims, wherein the suture actuator (10) is at least partially formed by at least one roll (31', 31").

8. The catheter device (1) according to any one of the preceding claims, wherein the suture actuator (10) is at least partially formed by a wire pusher (32) connected or connectable, at a distal end, to the suture (21).

9. The catheter device (1) according to any one of the preceding claims, wherein the suture actuator is at least partially formed by a gripper (35, 41), preferably a compressible hypotube (33), slidably arranged within the first lumen (4), and preferably the second lumen, adapted to releasably hold the first, and preferably the second, suture (21).

10. The catheter device (1) according to any one of the preceding claims, wherein the suture actuator (10) is at least partially formed by a magnetic actuator (32) adapted to interact, preferably selectively, with a magnetic element arranged on the suture (21).

11. The catheter device (1) according to any one of the preceding claims, wherein the suture actuator (10) is at least partially formed by a telescope mechanism (39, 40).

12. The catheter device (1) according to any one of the preceding claims, comprising a suture (21) and a spool (37) adapted to release the suture (21) in response to the suture actuator (10) being actuated, preferably wherein the spool (37) is arranged in a handle (5).

13. A suture (21) for use in a catheter device (1) according to any one of the preceding claims, having at least one of a magnetic element, a flare (36), and a high-friction surface.

14. The suture (21) according to claim 13, comprising a flat ribbon section, preferably wherein at least one marker (44, 44', 44") is arranged on a first and/or a second end of the flat ribbon section (43).

15. The suture (21) according to claim 14, wherein the marker is one of a color marker and a radio-opaque marker.

16. A method of suturing a tissue, preferably a heart tissue, comprising the step of piercing the tissue with a needle of a catheter device (1), preferably using a catheter device (1) according to any one of claims 1 to 12, and ejecting a suture (21) from the needle by means of a fluid flow.

17. The method according to claim 16, further comprising a step of capturing the suture (21), after ejection, using an expandable suture-capture mesh (205).

18. A suture actuator for a catheter device (1) according to any of claims 1 to 12, comprising a fluid reservoir (17) with a pre-loaded suture (21).
